# EUROPEAN PATENT SPECIFICATION

(11) **EP 3 896 676 B1**
(45) Date of publication and mention of the grant of the patent: **19.07.2023**
(21) Application number: 19895971.0
(22) Date of filing: 10.12.2019
(51) Int. Cl.: G09B 23/28, G09B 19/24, G09B 23/30, A41D 27/08, A43B 3/00, A43B 23/24, A61M 37/00, B32B 5/02, B32B 7/12, B32B 27/12, B32B 27/28

(54) **TATTOOABLE ELEMENT FOR DECORATING FASHION OR DECORATIVE OBJECTS AND HUMAN LIMBS**
TÄTOWIERBARES ELEMENT ZUM DEKORIEREN VON MODE ODER ZIERGEGENSTÄNDEN UND MENSCHLICHEN GLIEDMASSEN
ÉLÉMENT POUVANT ÊTRE TATOUÉ POUR ORNER DES OBJETS DE MODES OU DE DÉCORATION ET DES EXTRÉMITÉS DU CORPS HUMAIN

(30) Priority: 10.12.2018 ES 201831895 U
(43) Date of publication of application: 20.10.2021
(73) Proprietor: Drammeh Mora, Eddi, 08207 Sabadell (Barcelona) (ES); Moya Saez, Jordi, 03503 Benidorm (Alicante) (ES)
(72) Inventor: Drammeh Mora, Eddi, 08207 Sabadell (Barcelona) (ES); Moya Saez, Jordi, 03503 Benidorm (Alicante) (ES)
(74) Representative: Espiell Gomez, Ignacio
(86) International application number: PCT/ES2019/070838
(87) International publication number: WO 2020/120817

(56) References cited:
- WO-A1-2011/117722
- WO-A1-2011/117722
- WO-A1-2016/158884
- GB-A- 2 529 413
- GB-A- 2 529 413
- JP-A- 2006 195 447
- JP-B2- S6 210 553
- US-A1- 2014 308 651
- US-A1- 2015 351 499

## Description

### OBJECT OF THE INVENTION

The invention, as stated in the title of the present specification, relates to a tattooable element for decorating fashion or decorative objects that contributes advantages and features to the function intended, which are described in detail further on.

The object of the present invention relates, specifically, to a sheet element which, intended for being incorporated in fashion or decorative objects, such as footwear, items of clothing, fashion accessories, such as belts, bags, wallets, canvasses, tapestries, etc., and formed from a polymer layer attached to a reinforcing element such as a cloth or fibreglass base, where this latter part may or may not be part of the object itself, provides an ideal surface for being able to be tattooed, with any of the techniques conventionally used for directly tattooing skin, with optimal results in terms of execution and outcome, allowing for customised decoration, with any chosen image, of the object in which the element is implemented, either factory-made in that manner or as an added feature.

### FIELD OF APPLICATION OF THE INVENTION

The field of application of the present invention falls within the sector of the industry dedicated to the manufacture of fashion garments and objects, such as footwear, items of clothing, bags, etc., particularly focussing on the field of decorative elements for said type of objects.

### BACKGROUND OF THE INVENTION

As is known, tattoos are drawings etched onto the skin by means of a technique that involves the use of special needles to introduce colouring substances beneath the epidermis, providing a very specific and particular aesthetic result.

Synthetic skin or synthetic reproductions of parts of the body made from silicone for the practice of tattooing are also known. However, synthetic skin easily tears and as a consequence cannot be stitched.

The objective of the present invention is, therefore, to provide a means for allowing the incorporation of said type of images in fashion objects, such as footwear, items of clothing or others, or in decorative objects such as canvasses, tapestries, cushions, etc., so as to allow users to customise them with tattoos chosen by said users.

However, applying this same technique on surfaces other than the skin requires the development of a special surface which allows the tattoo to be executed in a suitable manner, for which purpose the element of the present invention has been developed.

US2015351499 is considered the closest prior art. It shows an article with a polymeric component where ink can be injected and remain embedded. The component has several layers.

GB2529413 discloses a multi layered tattoo practice pad comprising layers of silicon and silicone gel. It is designed for learning the craft and controlling the depth of the needle insertion. An artificial skin, also for learning to tattoo, is made known in WO2011117722. It comprises two layers that simulate the texture and consistency of human skin epidermis, and the dermis and hypodermis of human skin.

### DESCRIPTION OF THE INVENTION

The tattooable element for decorating fashion or decorative objects proposed by the invention is configured as an optimal solution, since, based on its implementation, the aforementioned objectives are satisfactorily and unquestionably achieved, with the characterising details making it possible and suitably distinguishing it being included in the final claims attached to the present description.

More specifically, what the invention proposes, as indicated above, is a sheet element designed as an element to decorate, presenting the particularity that it is tattooable, in addition to being susceptible to being handled, sewn and stitched, where it can be implemented in the industrial production system for any type of footwear (shoes, trainers, boots, etc.) and/or any type of item of clothing (jackets, pullovers, blazers, T-shirts, pants, etc.) or other fashion accessories (belts, bags, wallets, etc.) or decorative objects (canvasses, chair or sofa tapestries, cushions, etc.), or as an accessory that can be sold as an element that is separate and can be fixed to any of said objects once they have been manufactured and already existing on the market.

To that end and more specifically, the decorative tattooable element is essentially configured from a polymer layer suitable for being tattooed and a reinforcing element of the polymer layer.

This reinforcing element improves the features of the polymer layer allows the handling, sewing and stitching of the tattooable element, allowing same to be incorporated in the fashion object or article of clothing in question, even constituting the actual fabric for producing the article of clothing or being a part thereof.

Additionally and in the event of the polymer layer tearing, the reinforcing element allows the pieces of the polymer layer to remain in their original position, thus preventing the figure of the tattoo from losing its original shape.

In a preferred embodiment, the reinforcing element is an element that is separate from the polymer layer and fixed to the polymer layer.

The reinforcing element can be a fabric or fibreglass. Both the fabric and the fibre limit the elastic properties of polymers such as silicone, thus allowing them to be susceptible to stitching and implemented in fashion and/or decorative objects.

The reinforcing element can be fixed to the polymer layer by means of glue or fusing by applying heat and pressure on the reinforcing element against the polymer layer.

Alternatively, in another preferred embodiment the reinforcing element is integrated into the polymer layer.

Preferably, the reinforcing element is a mesh integrated into the polymer layer or an array of fibres integrated into the polymer layer. The advantage is that when the polymer layer is fused with the fabric or fibre mesh, both fused elements work like a single piece, adding resistance to the stitching and increasing the durability in other fields of application.

Regardless of how the reinforcing element is carried out, the polymer layer is preferably made of silicone. Silicone offers a smooth surface suitable for being tattooed with optimal results in terms of definition, durability and artistic execution.

Regardless of how the reinforcing element is carried out, the polymer layer preferably has a thickness of more than 1 mm, and even more preferably a thickness of more than 3 mm.

In any case, the main advantage of the tattooable element for decorating fashion or decorative objects provides, from a commercial approach, a technology that is very attractive in terms of value, according to current trends, for customising and adding a distinctive quality to footwear, articles of clothing or other fashion accessories.

### DESCRIPTION OF THE DRAWINGS

To complete the description provided herein, and for the purpose of helping to make the features of the invention more readily understandable, this description is accompanied by drawings constituting an integral part of the same, which by way of illustration and not limitation represents the following:
Figure number 1 shows a schematic perspective view of the two sheet layers forming the tattooable element for decorating fashion or decorative objects, object of the invention, represented in an exploded view;
Figure number 2 shows a schematic section view of the tattooable element according to the invention, in this case represented once the two sheet layers it comprises have been attached to one another;
Figure number 3 shows a schematic perspective view of the tubular-shaped tattooable element;
Figure number 4 shows a schematic perspective view of the tubular-shaped tattooable element fixed on a user's forearm;

### PREFERRED EMBODIMENT OF THE INVENTION

In view of the aforementioned figures, and in accordance with the adopted numbering, one may observe therein a non-limiting exemplary embodiment of the proposed tattooable element for decorating fashion or decorative objects, comprising what is described in detail below.

Thus, as can be observed in said figures, the element (1) in question is essentially configured from a sheet element formed by two layers of different material: an upper silicone layer (2), providing the smooth surface suitable for tattooing with any conventional technique, and preferably having a thickness of about 1 mm, and a lower layer (3) acting as a base for the upper layer (2), where said lower layer (3) may be made of either fabric or fibreglass.

Furthermore, the upper silicone layer (2) is preferably made of medical grade silicone (RTV (*Room Temperature Vulcanisation*)), since in addition to being hypoallergenic, it does not contain latex or toxins, or any type of additive, nor is it porous, whereby its use in articles of clothing in contact with the skin is feasible, and especially, it provides an ideal surface for tattooing, with optimal results of definition and resistance.

It should be pointed out that the lower base layer (3) is preferably attached to the upper silicone layer (2) by means of any fixing system, for example a glue layer (4), which may be fused with same, for example, by means of applying heat.

Furthermore, the element (1) may be trimmed in order to give it the desired shape, and coupled to the object by means of stitching, gluing, or any other fixing means, even by means of a removable system in order to take it off and put it on at will.

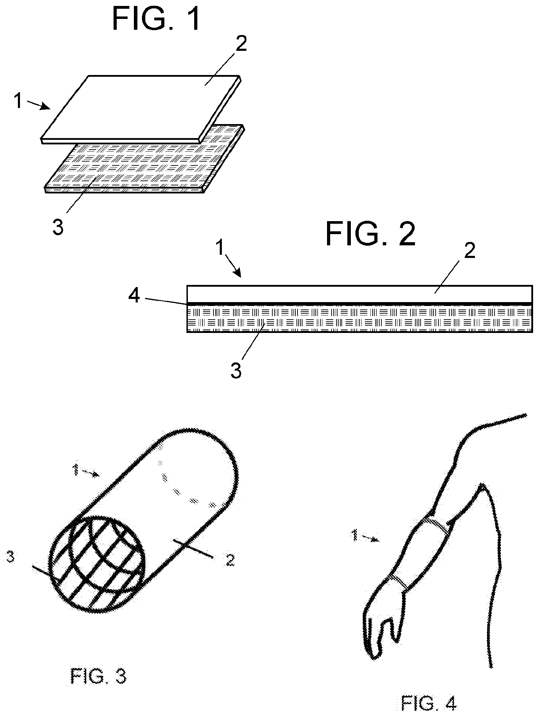

In a preferred embodiment, the tattooable element is tubular-shaped. In an even more preferred embodiment, the tattooable element is elastic. This embodiment allows the tattooable element to be fixed on a user's body, for example on the forearm as shown in Figure 4, allowing the tattoo artist to have a tattooing experience very similar to the real experience.

In a preferred embodiment, the reinforcing element (3) prevents the needle from penetrating the tattooable element during the process of tattooing the polymer layer (2). The user can thereby be tattooed without any apprehension about the needle penetrating the tattooable element and being able to damage human skin.

## Claims

1. The tattooable element for decorating fashion or decorative objects, comprising a polymer layer (2) suitable for tattooing **characterized in that** a reinforcing element (3) made of a fabric or fibreglass is fixed to the polymer layer (2) by means of glue or fusing.

2. The tattooable element according to claim 1, **characterised in that** reinforcing element (3) is integrated within the polymer layer (2).

3. The tattooable element according to claim 2, **characterised in that** the reinforcing element (3) is a mesh.

4. The tattooable element according to claim 1, **characterised in that** the reinforcing element (3) is fibres.

5. The tattooable element according to any of the preceding claims, **characterised in that** the polymer layer (2) is made of silicone.

6. The tattooable element according to any of the preceding claims, **characterised in that** the polymer layer (2) has a thickness of more than 1 mm.

7. The tattooable element according to claim 6, **characterised in that** the polymer layer (2) has a thickness of more than 3 mm.

8. The tattooable element according to any of the preceding claims, **characterised in that** the reinforcing element (3) prevents the needle from penetrating the tattooable element during the process of tattooing the polymer layer (2).

9. The tattooable element according to any of the preceding claims, **characterised in that** it is tubular-shaped.

10. The tattooable element according to any of the preceding claims, **characterised in that** it is elastic and allows being adapted to the support on which the tattooable element is fixed.

## Patentansprüche

1. Tätowierbares Element zum Verzieren von Mode- oder Dekorationsgegenständen, das eine zum Tätowieren geeignete Polymerschicht (2) umfasst, **dadurch gekennzeichnet, dass** ein Verstärkungselement (3) aus Textil oder Glasfaser an der Polymerschicht (2) durch Kleben oder Schmelzen befestigt ist.

2. Tätowierbares Element nach Anspruch 1, **dadurch gekennzeichnet, dass** das Verstärkungselement (3) in die Polymerschicht (2) integriert ist.

3. Tätowierbares Element nach Anspruch 2, **dadurch gekennzeichnet, dass** das Verstärkungselement (3) ein Geflecht ist.

4. Tätowierbares Element nach Anspruch 1, **dadurch gekennzeichnet, dass** das Verstärkungselement (3) aus Fasern besteht.

5. Tätowierbares Element nach einem der vorhergehenden Ansprüche, **dadurch gekennzeichnet, dass** die Polymerschicht (2) aus Silikon besteht.

6. Tätowierbares Element nach einem der vorhergehenden Ansprüche, **dadurch gekennzeichnet, dass** die Polymerschicht (2) eine Dicke von mehr als 1 mm aufweist.

7. Tätowierbares Element nach Anspruch 6, **dadurch gekennzeichnet, dass** die Polymerschicht (2) eine Dicke von mehr als 3 mm aufweist.

8. Tätowierbares Element nach einem der vorhergehenden Ansprüche, **dadurch gekennzeichnet, dass** das Verstärkungselement (3) verhindert, dass die Nadel während des Tätowierprozesses der Polymerschicht (2) in das tätowierbare Element eindringt.

9. Tätowierbares Element nach einem der vorhergehenden Ansprüche, **dadurch gekennzeichnet, dass** es rohrförmig ist.

10. Tätowierbares Element nach einem der vorhergehenden Ansprüche, **dadurch gekennzeichnet, dass** es elastisch ist und eine Anpassung an den Träger ermöglicht, auf dem das tätowierbare Element befestigt ist.

## Revendications

1. Élément tatouable pour la décoration d'objets de mode ou de décoration, comprenant une couche de polymère (2) apte au tatouage **caractérisé en ce qu'**un élément de renforcement (3) constitué de tissu ou fibre de verre est fixé à la couche de polymère (2) au moyen de colle ou de fusion.

2. Élément tatouable selon la revendication 1, **caractérisé en ce que** l'élément de renforcement (3) est intégré à l'intérieur de la couche de polymère (2).

3. Élément tatouable selon la revendication 2, **caractérisé en ce que** l'élément de renforcement (3) est un treillis.

4. Élément tatouable selon la revendication 1, **caractérisé en ce que** l'élément de renforcement (3) est des fibres.

5. Élément tatouable selon l'une quelconque des revendications précédentes, **caractérisé en ce que** la couche de polymère (2) est en silicone.

6. Élément tatouable selon l'une quelconque des revendications précédentes, **caractérisé en ce que** la couche de polymère (2) a une épaisseur supérieure à 1 mm.

7. Élément tatouable selon la revendication 6, **caractérisé en ce que** la couche de polymère (2) a une épaisseur supérieure à 3 mm.

8. Élément tatouable selon l'une quelconque des revendications précédentes, **caractérisé en ce que** l'élément de renforcement (3) empêche l'aiguille de pénétrer dans l'élément tatouable lors du processus de tatouage de la couche de polymère (2).

9. Élément tatouable selon l'une quelconque des revendications précédentes, **caractérisé en ce qu'**il est de forme tubulaire.

10. Élément tatouable selon l'une quelconque des revendications précédentes, **caractérisé en ce qu'**il est élastique et permet de s'adapter au support sur lequel est fixé l'élément tatouable.
